# EUROPEAN PATENT SPECIFICATION

(11) **EP 0 509 012 B1**
(45) Date of publication and mention of the grant of the patent: **19.07.1995**
(21) Application number: 91901856.4
(22) Date of filing: 09.01.1991
(51) Int. Cl.: A61F 13/15

(54) **COVERSTOCK**
DECKUNGSSCHICHTEN
FILM DE COUVERTURE

(30) Priority: 10.01.1990 GB 9000573
(43) Date of publication of application: 21.10.1992
(73) Proprietor: SMITH & NEPHEW plc, London WC2R 3BP (GB)
(72) Inventor: CARTER, Andrew, James, Saffron Walden Essex CB11 4AL (GB)
(74) Representative: Gilholm, Stephen Philip
(86) International application number: GB9100026
(87) International publication number: WO9110415

(56) References cited:
- EP-A- 0 052 403
- EP-A- 0 156 471
- EP-A- 0 203 823
- FR-A- 2 241 265
- FR-A- 2 252 817
- FR-A- 2 397 797
- FR-A- 2 400 887

## Description

The present invention relates to coverstocks suitable for use as a cover for absorbent devices such as sanitary napkins, incontinence pads and surgical dressings. The present invention also relates to absorbent devices containing such coverstocks and to their manufacture.

It is desirable that body fluid such as blood, urine or wound exudate entering the absorbent material of an absorbent device such as a sanitary napkin, diaper, bed pad, incontinence pad, surgical dressing or bandage should be dispersed throughout the absorbent material in order to utilise its full absorptive capacity. It is undesirable feature of current absorbent device design that absorbed fluid may re-emerge at the entry point or at sites distant from the entry pint when localised pressure is brought to bear on the absorbent device. This phenomenon of the re-emergence of absorbed fluid is commonly termed 'wet-back'.

Generic document FR-A-2397797 discloses an absorbent protective article comprising an absorption layer which absorbs liquids, a layer at the back of the article that is permeable to liquids and a covering layer formed by a foil provided with a large number of recesses that extend into the absorption layer, wherein each recess is provided with one hole for the passage of liquids during the absorption process.

FR-A-2252817 discloses a disposable absorbent article comprising a hydrophobic non fibrous film for contacting the body which film comprises a plurality of openings which have the role of valves. The openings are capable of allowing the passage of at least 20mls of liquid pass through a 130cm² surface in ten seconds and can substantially prevent the passage of liquid in the reverse direction.

It has been found that by using as a coverstock a water permeable, apertured, contoured, polymeric film formed from elastomeric polymer and in which the ratio of apertures to depressed areas of greater than one, an absorbent device may be manufactured which has good absorbent and wet-back properties. Such films and absorbent products made therefrom are described in International Patent Publication No. WO 87/01029.

The apertured films described therein are contoured, for example by embossing, to provide depressions having a generally square or hexagonal outline or shape. The area between the depressions being a series of interconnected generally straight and parallel sided ridges.

Such coverstocks, although possessing the necessary good wet-back properties, may lack certain aesthetic properties such as softness to the touch and a pleasing appearance. Because such coverstocks are made of synthetic polymeric materials, they have a tendency to look and feel like 'plastics' materials, a propensity which can be aesthetically unpleasing to the user of absorbent devices such as sanitary towels which have a body contacting layer of such a coverstock.

We have now found that the softness or hand of such coverstocks may be considerably improved, without adversely affecting wet-back properties by imparting depressions in the film wherein the perimeter is curved.

The present invention therefore provides a coverstock suitable for use in an absorbent device which comprises a film having a multiplicity of depressed areas, wherein each depressed area contains at least one aperture and essentially all of the depressed areas have curved perimeters; characterised in that:-
(i) the film has more apertures than depressed areas
(ii) the apertures are in the depressed areas and in the ares between the depressed areas, and
(iii) the area between the depressed areas is 22-50% of the area of the film.

By the expression 'curved perimeter' is meant a perimeter which is essentially free from straight linear portions. Examples of depressed areas having curved perimeters are circular, ellipsoidal or pear shaped depressions. The perimeter of the depression may be regular, irregular, simple or complex. The shape may be a simple geometric shape such as a circle or ellipse or a more complex shape such as a lobate design eg. a floral pattern. The depressed areas may be of differing shapes and sizes to make up a larger pattern or design.

Suitably the apertures may be distributed throughout the depressed areas in a random manner. However, it is possible but less preferred that by judicious choice of the frequency of the apertures and the depressed areas, a coverstock in which the apertures are distributed in a regular manner throughout the depressed areas can be achieved.

Within the scope of the present invention are coverstocks in which the depressed areas may contain different numbers of apertures for example some depressed areas may contain one aperture and some depressed areas may contain more than one aperture but the ratio of the total number of apertures to the total number of depressed areas will be greater than unity.

Suitably each depressed area will contain at least one aperture and aptly the depressed areas will contain an average of more than one aperture per depressed area. Generally, the ratio need not exceed 8:1 and suitably will be from 3 to 6:1 and preferably will be from 4 to 5:1.

The area between the depressed areas of the coverstock will also contain apertures. The distribution of apertures per unit area will suitably be the same for both the non-depressed and depressed areas. However, if desired there may be a disproportionate distribution of the apertures between the depressed and non-depressed areas on a unit area basis. For example the depressed areas may contain more depressions per unit area than in the non-depressed areas. Alternatively the non-depressed areas may have a higher ratio of apertures than the depressed areas.

The depressions may be regularly or randomly disposed over the whole of the film surface. Regularly arranged depressions may be in the form of parallel but spaced apart lines of depressions. However, it is preferred to dispose the depression either randomly or in an offset regular pattern ie. each depression is offset in its disposition with respect to adjacent depressions in either or both the longitudinal (machine) direction or lateral (transverse) direction of the film, in order to improve the visual appearance of the finally formed coverstock.

Suitably the depressed areas of the coverstock may number greater than 4, suitably greater than 10 depressions per sq. cm. Generally the number need not exceed 40, and may be less than 35 depressions areas per sq. cm. Preferably the number will be from 15 to 30 depressions per sq. cm.

The specified ratio of depressed areas to non-depressed areas has been found to provide a desirable softness of the coverstock to touch. The greater the area of non-depressed areas within the specified range the softer will be the touch or hand of the coverstock. The size and numbers of the depressed areas will be such that at least 22% of the area of the coverstock is made up of non-depressed areas. It has been found that if the area of the coverstock between the depression areas exceeds 50% the wet-back properties of the coverstock will be adversly affected. Aptly, the coverstocks will have non-depressed areas of greater than 25% of the film surface. Generally the non-depressed areas will suitably be not more than 40% preferably from 27 to 37%, and typically may be about 30%.

Coverstocks having non-depressed areas between the depressed areas within the specified range may be produced by selection of the geometry, dimensions and position of the array of the elements which are used to form the depressions in the apertured film. The areas within the specified range include the area of voids formed by the apertures which are located within the non-depressed areas.

In the formed coverstocks of the invention, the areas between the depressed areas can be determined by, for example, the following method. Indian ink is spread on a ground glass plate and excess ink scraped off using the edge of a standard microscope slide. A sample of the coverstock is placed on the ink surface with the side bearing the depressions facing towards the ink coating. A microscope slide is then placed on top of the sample. The microscope slide is removed from the coverstock. The coverstock is then removed from the coated glass plate and the surface in contact with the ink examined. The area coated by ink from the glass plate represents the area between the depressed areas of the coverstock.

Suitably the coverstock may contain at least 8 apertures per sq. cm and generally will contain upto about 300 apertures per sq. cm. More suitably the coverstock will contain more than 15 per sq. cm and may contain from 100 to 200 apertures per sq. cm.

Suitably the coverstock may have depressions which have at least two apertures in the wall of each depression. More suitably it will have upto 10 apertures per depression and preferably upto 4 apertures per depression.

Aptly the area of each aperture will be at least 0.01 sq. mm. and may be upto 1 sq. mm. More favourably the area will be at least 0.1 sq. mm. and preferably the area will be from 0.25 to 0.75 sq. mm.

Suitably the total open area of the apertured film may comprise at least 5% of the area of the film and aptly may be upto 50% of the total area of the film. The selection of the desired open area may depend upon the use intended for the final contoured apertured film. Thus, for example where the intended use is as a wound facing layer in a dressing the open area may be not more than 15%, for example from 5 to 15%, whereas when used as a cover layer for a sanitary towel or diaper the open area may be as high as 40% eg. from 15 to 40%.

Suitable polymers for the film include polyether ester, polyurethanes, styrene-butadiene and styrene-isoprene block copolymers, polyisobutadiene and blends of ethylene-vinyl acetate copolymers with polyolefins for example with polystyrene.

Polymeric materials which are suitable for preparing the contoured apertured film of the invention include thermoplastic elastomeric polymers or polymer blends. A favoured material is a blend of an ethylene-vinyl acetate copolymer (EVA) preferably in amounts of at least 10% by weight and an incompatible polymer preferably in amounts of at least 10% by weight such as a polyolefin. Examples of suitable polyolefins include polystyrene and polyethylenes such as low density and linear low density polyethylenes. A particularly preferred material is a blend of from 10 to 90 parts by weight of ethylene-vinyl acetate copolymer and 90 to 10 parts by weight of polyolefin and more preferably 20 to 80 parts by weight of ethylene-vinyl acetate copolymer and 80 to 20 parts by weight of polystyrene. Typical examples of such a blend comprises a blend of 40 parts ethylene-vinyl acetate copolymer and 60 parts high impact polystyrene, or a blend about 25 parts ethylene-vinyl acetate copolymer, about 13 parts high impact polystyrene and about 60 parts linear low density polyethylene.

If necessary the polymeric material may include up to 10% of fillers or whitening agents such as titanium dioxide. The material may desirably contain upto about 5% by weight of suitable surfactant, for example about 1% by weight.

Other materials suitable for preparing the coverstocks of the invention includes elastomeric polyether ester block copolymers such as Hytrel 4056 (Trade Mark).

Other suitable materials include polyether polyamide block copolymers such as those which are designated Pebax (Trade Mark). A suitable copolymer is Pebax 2533 SN 00.

The polymer film used to form the coverstock of the invention is usually formed by conventional extrusion methods of the polymer or polymer blend. Suitably the thickness of the film when extruded is from 25 to 200»m and more suitably is 30 to 150»m thick and preferably from 35 to 10»m thick. The thickness will depend to some extent on the properties of the film as regards strength and softness. The coverstock when used in a sanitary protection device or a surgical dressing where it contacts the skin should be soft whilst at the same time being strong enough to be worn without tearing.

The coverstock itself will have a thickness as measured from the level of the plane of the bottom of the depressed area of the film to the top of the area between depressions. Suitably this thickness or depth of depressed area is at least 0.2mm. Aptly the depth may be upto 3mm and more suitably upto 1mm for example from 0.5 to 1mm. and preferably is about 0.7mm. The coverstock when used in an absorbent device therefore provides separation of the absorbent from the wearer.

The coverstocks of the present invention are particularly suitable for use in absorbent devices. Absorbent devices when used herein include sanitary napkins, diapers, incontinence pads, and in a somewhat different area, surgical dressings. In these devices the coverstock comprises at least a part of the surface which may at some time in use contact the skin of the wearer. The coverstock normally provides a dry surface and when placed under pressure serves to restrict passage of absorbed fluid whereby the wearer's skin remains in a substantially dry condition. The depressions are arranged to face away from the body of the wearer and towards the absorbent material.

Wet-back may be expressed as the amount of absorbed fluid represented to the surface of the coverstock and can be determined by the following method.

The coverstock is placed on top of a bat of absorbent material eg. wood pulp fibres, with the depressed areas facing upwardly. A predetermined amount of a 1% saline solution is delivered slowly (eg. dropwise) at a predetermined rate onto the centre of the coverstock. Ten seconds after cessation of the saline addition 20 preweighed Whatman No 1 filter papers are placed on top of the coverstock and a 2kg weight (base area 5 x 10cm.) placed on top of the filter papers. After one minute the weight is removed and the filter papers reweighed. The difference between the original weight of the filter papers and the weight after removal from the coverstock is the wet-back value. Coverstocks having satisfactory wet-back properties would have wet-back values by the above test, of less than 0.5gm.

Coverstocks of the present invention preferably have wet-back values of less than 0.2gm.

In a second aspect of the present invention there is provided an absorbent device comprising an absorbent material and a coverstock as herein before defined.

Suitably the absorbent device is in the form of a sanitary napkin or diaper in which at least the body facing surface of the device includes a coverstock of the invention. Suitably the whole of the body facing surface comprises a coverstock. Most aptly the device is a sanitary napkin. The sanitary napkin may have the conventional structure which the skilled worker would recognise as usual in the art, that is a liquid pervious body facing layer, absorbent material and a fluid impermeable backing layer. The facing layer and fluid impermeable backing layer may be heat sealed around their edges to enclose the absorbent material.

Alternatively the coverstock may extend around the whole of the absorbent material to overlap on the garment facing side and the edges adhered by for example a hot melt or pressure sensitive adhesive. In this embodiment a fluid impermeable layer may be placed at least between the garment facing side of the absorbent and coverstock. In yet a further alternative the coverstock may form a strip in part of a larger piece of film whereby the depressed and apertured portions form the body contacing surface and the adjacent non-apertured and optionally non-depressed portions of the coverstock are wrapped around the remainder of the absorbent material to provide the fluid impermeable wrapper.

The absorbent material used in the invention can be any of the absorbent layers used in conventional hygienic absorbent pads. Suitable absorbent materials for such layers include, comminuted/fluffed wood pulp, carded cotton webs, viscose rayon fibres, tissue wadding, grafted cellulose super absorbents, polymeric super absorbents or mixtures thereof. The absorbent layer can optionally contain an insert such as a tissue wadding to aid fluid distribution within the layer.

In a further embodiment of this aspect of the invention the absorbent device may be in the form of a surgical dressing.

The coverstock may be prepared as follows: a film is formed from a polymeric material by, for example, conventional blending and/or extrusion methods. The film may also contain fillers, whiteners, plasticisers, surfactants and the like to aid processing and to provide satisfactory surface characteristics and appearance to the finished film. For example, a polymer blend containing about 25 parts by weight of ethylene-vinyl acetate copolymer (containing 18% vinyl acetate content), 59 parts by weight of linear low density polyethylene, 13 parts by weight of polystyrene, 3% by weight of the polymer of titanium dioxide and 0.7% by weight of a lauric diethanol amide surfactant (Lankrostat JP) can be formed by mixing the polymers in a blade mixer heated to 165°C for 4 minutes. The resultant mix can be removed from the mixer and formed into a sheet using a heated roll mill. After cooling the sheet can then be granulated in a Masson cutter.

Using, for example, a Brabender Extrusiograph Extruder (length to diameter screw ratio of 25:1) driven by a Brabender PLE 651 Plasticorder, the mixture may be extruded through a 150mm film die into a nip of a two roller casting unit placed near the die. In this manner a resultant film having a mass weight per unit area of about 40gsm can be produced.

The film may then be apertured during or after formation using the normal methods used in the art including flame perforation and pin moulds with heated or non-heated pins. In one method of the invention the film may be placed against a pin mould and covered by a plain polyethylene film. The thus formed three layer sandwich is then compressed whereupon the pins of the pin mould aperture the film removing a piece of that film have the area of the pin. These pieces of film adhere to the polyethylene film. On removal of the compressive force, the polyethylene film along with small pieces of film are then removed to reveal a substantially flat apertured film of the polymer.

Alternatively an apertured film may be provided with depressions by pressing against a roller or a plate carrying a pattern of raised areas surrounded by trough of flat areas. The raised areas which give rise to the depressed contours on the coverstock may be of any curved shape but raised areas which are in the form of circular or elipsoidal truncated cones are preferred. Lobate or irregularly shaped depressed areas may also be formed. The pattern of areas impressed in the apertured film and the frequency of apertures may be arranged so that any depression may contain one or more than one aperture, indeed some of the apertures will fall in the land areas between the depressed areas in the film. This gives a random appearance to the distribution of the apertures amongst the depressed areas of the film even though such distribution may have arisen by impressing a regular pattern of depressions on a regular pattern of apertures in the film. Aptly each depression will contain at least one aperture. In an apt form the pattern of depressions and the frequency of apertures may give rise to a substantially regular distribution of apertures amongst the depressed areas in which there are 2 to 10 apertures in each depressed area.

In one embodiment of the invention the apertured film may be contoured by placing it on the surface of a thermoplastic film, such as a polypropylene film, which has discrete raised areas for example 14 raised areas per sq. cm. The raised areas may be offset with respect to the adjacent raised areas and may be coated with a silicone release agent to facilitate removal of the formed contoured apertured film. The raised areas may be of any circular shape. The apertured film is contoured by compressing it against the raised areas of the film. Usually a resilient material, such as a foam, is present on the other side of the apertured film. The compression may be provided by a conventional press or by passing between the nip of a two rollers. It is possible that additional apertures may be placed in the already apertured film at this stage. These additional apertures are formed in the film at the tip of each of the raised areas in the thermoplastic film. The conditions of temperature and pressure used in the process will depend upon the properties of the polymeric film employed. After this treatment the contoured apertured film may be peeled off the thermoplastic film. The size of the depressions or embossments and the pattern of bosses employed will vary depending upon the size of apertures in the elastomeric film. The depressed areas in the film may be adapted in size so that at least two apertures fall in the walls of each of the depressions formed in the apertured film during the compression process.

In a further embodiment the apertured film may be contoured by passing it between two rollers one or both of which of have a pattern of raised and/or depressed areas on their surface. The rollers may be heated to a suitable temperature to assist in contouring the film. In one method only one roller carries a pattern of raised areas on its surface. The other roller, the smooth roller, preferably has a soft resilient surface of rubber or foam which allows the raised areas on the other roller, which is normally made of hard material such as metal, to press into and permanently deform the apertured film. A preferred method is to use two rollers which have an intermeshing pattern of raised and depressed areas on their surface.

A further object of the present invention is to provide a process for the preparation of a coverstock which process comprises forming apertures in a film of a polymer and then compressing the apertured film against a pattern of raised and/or depressed areas whereby a pattern of depressions is impressed in the apertured film, the number of apertures and depressions being such that the ratio of apertures to depressions is greater than unity.

Thus in accordance with this further aspect there is provided a process for the production of a coverstock characterised in that the process comprises compressing a film having a multiplicity of apertures against a pattern of depressions is impressed in the apertured film, the number of apertures and depressions being such that
(i) each depressed area of the coverstock contains at least one aperture,
(ii) the apertures are in both the depressed areas and in the areas between the depressed areas of the coverstock
(iii) essentially all of the depressed areas have curved perimeters and
(iv) the area between the depressed areas of the covestock is 22 to 50% of the area of the film.

### Example 1

A coverstock was produced by embossing an film containing 103 diamond shaped apertures per sq. cm. Each aperture was 0.66mm. long by 0.35mm. wide and the total open area of the film was 36%. The film was formed from a polymer blend of 25 parts by weight of Ethylene-Vinyl Acetate Copolymer (18% vinyl acetate, 59 parts by weight Linear Low Density Polyethylene and 13 parts by weight High Impact Polystyrene), mixed with 0.6% by weight Lauric Diethanol Amide (Surfactant) and 3% by weight titanium dioxide.

The apertured film was heated and softened by blowing a current of hot air at 120°C to 150°C onto the film. Thereafter the heated film was passed into the nip of two embossing rollers. The cylindrical surface of one roller was smooth and made of a resilient rubber. The surface of the other roller was studded with metal protruberances (12 per 2.54 cm or linear inch). Each protruberance was of right circular conical shapes having a nominal base diameter of between 1.0 and 1.7 mm. and a depth of 0.75 mm. to provide an embossed product having an area formed between depressions of 29% of the total film area.

During the heating step, prior to embossing slight shrinkage of the apertured film occured. The resulting embossed coverstock product had a total open area of 29%, the apertures had a nominal size of 0.6mm. long by 0.3mm. wide and the aperture density increased to 164 apertures per cm².

The wet-back value for this coverstock was 0.03gm.

In the manufacture of an absorbent device, an absorbent core (width 60mm, length 216mm) of comminuted fluff pulp (6.6g), having a central layer insert of folded tissue wadding can be placed centrally onto a strip of the coverstock (width 70mm, length 240mm) so that the openings in the depression would be in contact with the absorbent core. A liquid impervious film of polyethylene can then be placed over the other side of the absorbent core and heat sealed around its edges to the apertured film so that the absorbent core is sandwiched between the two films.

A portion or portions of the outer facing surface of the liquid impervious film may carry a pressure sensitive adhesive layer covered by a release paper whereby in use the absorbent device in the form of a sanitary napkin may be adhered to a garment of the wearer.

### Example 2

An apertured film produced from a polymer blend containing 40 parts by weight of ethylene-vinyl acetate copolymer (containing 18% vinyl acetate content), 40 parts by weight of linear low density polyethylene, 20 parts by weight of high impact polystyrene, 3% by weight of the polymer of titanium dioxide and 0.6% by weight of Lauric Diethanol Amide (Surfactant) and containing 90-100 ellipsoidal apertures per sq. cm which were, having an aperture area of 0.27 sq. mm. and a film open area of 23% was formed into a coverstock by the following method:
The apertured film was placed against a layer of a non-woven fabric known as Novelin (available from J.W. Suominen) and the non-woven fabric side of the thus formed sandwich laid against the surface of a polypropylene sheet, embossed with random pattern (average of 3.5/cm) of raised discrete, conical bosses. A resilient polyurethane foam was placed over the apertured film, and the resulting sandwich was subjected to pressure and heat, 80°C in a press for 5 minutes. After removal of the sandwich of layers from the press, the embossing sheet, foam and the non-woven fabric were removed from the contoured apertured film.

The formed coverstock had about 20 depressions per sq. cm, a weight per unit area of approximately 40 g/m², and the non-depressed areas of the coverstock were about 28% of the total area.

## Claims

1. A coverstock suitable for use in an absorbent device which comprises a film having a multiplicity of depressed areas, wherein each depressed area contains at least one aperture and essentially all of the depressed areas have curved perimeters; characterised in that:-
(i) the film has more apertures than depressed areas
(ii) the apertures are in the depressed areas and in the areas between the depressed areas, and
(iii) the area between the depressed areas is 22-50% of the area of the film.

2. A coverstock as claimed in claim 1 wherein the perimeter of the depressed area is essentially circular or ellipsoidal.

3. A coverstock as claimed in claim 1 or claim 2 wherein the depressions are lobate.

4. A coverstock as claimed in claim 1 wherein the depressed areas contain an average of more than one aperture per depressed area.

5. A coverstock as claimed in any one of the preceding claims wherein the depressed areas contain upto 8 apertures per depressed area.

6. A coverstock as claimed in any one of the preceding claims wherein the areas between the depressed areas form 25 to 40% of the area of the film.

7. A coverstock as claimed in any one of the preceding claims containing from 4 to 40 depressions per sq. cm. of film.

8. A coverstock as claimed in any one of the preceding claims wherein the apertures are distributed substantially equally over both the depressed areas of the film and the areas between the depressions.

9. A coverstock as claimed in any one of the preceding claims containing from 8 to 300 apertures per sq. cm.

10. A coverstock as claimed in any one of the preceding claims wherein each aperture has an area of from 0.01 sq. mm. to 1.0 sq. mm.

11. A coverstock as claimed in any one of the preceding claims wherein the total open area of the film is from 5 to 50% of the area of the film.

12. A coverstock as claimed in any one of the preceding claims wherein the depth of the depressed area is from 0.2 to 3.0 mm.

13. A coverstock as claimed in any one of the preceding claims wherein the depressed areas are of different sizes.

14. A coverstock as claimed in any one of the preceding claims wherein the depressed areas are disposed in an offset array with respect to each other.

15. A coverstock as claimed in any one of the preceding claims wherein the depressed areas are disposed in a random array.

16. An absorbent device comprising a coverstock as claimed in any one of the preceding claims in combination with an absorbent material.

17. A process for the production of a coverstock characterised in that the process comprises compressing a film having a multiplicity of apertures against a pattern of raised and/or depressed areas whereby a pattern of depressions is impressed in the apertured film, the number of apertures and depressions being such that
(i) each depressed area of the coverstock contains at least one aperture,
(ii) the apertures are in both the depressed areas and in the areas between the depressed areas of the coverstock
(iii) essentially all of the depressed areas have curved perimeters and,
(iv) the area between the depressed areas of the coverstock is 22 to 50% of the area of the film.

## Patentansprüche

1. Zur Verwendung an einem absorbierenden Mittel geeignetes Abdeckmaterial, das einen Film mit einer Vielzahl von eingedrückten Bereichen aufweist, wobei jeder eingedrückte Bereich wenigstens eine Öffnung enthält und im wesentlichen alle eingedrückten Bereiche eine gekrümmte äußere Begrenzung haben, dadurch gekennzeichnet, daß:
(i) der Film mehr Öffnungen als eingedrückte Bereiche hat
(ii) die Öffnungen in den eingedrückten Bereichen und in den Bereichen zwischen den eingedrückten Bereichen angeordnet sind
(iii) der Bereich zwischen den eingedrückten Bereichen 22-50% der Fläche des Filmes ausmacht.

2. Abdeckmaterial nach Anspruch 1, bei welchem die äußere Begrenzung des eingedrückten Bereiches im wesentlichen kreisförmig oder elliptisch verläuft.

3. Abdeckmaterial nach Anspruch 1 oder 2, bei welchem die Eindrückungen gelappt sind.

4. Abdeckmaterial nach Anspruch 1, bei welchem die eingedrückten Bereiche im Durchschnitt mehr als eine Öffnung pro eingedrücktem Bereich enthalten.

5. Abdeckmaterial nach einem der vorhergehenden Ansprüche, bei welchem die eingedrückten Bereiche bis zu 8 Öffnungen pro eingedrücktem Bereich enthalten.

6. Abdeckmaterial nach einem der vorhergehenden Ansprüche, bei welchem die Bereiche zwischen den eingedrückten Bereichen 25 bis 40% der Fläche des Filmes ausmachen.

7. Abdeckmaterial nach einem der vorhergehenden Ansprüche, welches zwischen 4 bis 40 Eindrückungen pro cm² Film hat.

8. Abdeckmaterial nach einem der vorhergehenden Ansprüche, bei welchem die Öffnungen im wesentlichen gleich über die eingedrückten Bereiche des Filmes und die Bereiche zwischen den Eingedrückungen verteilt sind.

9. Abdeckmaterial nach einem der vorhergehenden Ansprüche, welches 8 bis 300 Öffnungen pro cm² enthält.

10. Abdeckmaterial nach einem der vorgehenden Ansprüche, bei welchem jede Öffnung eine Ausdehnung von 0,01 mm² bis 1,0 mm² hat.

11. Abdeckmaterial nach einem der vorhergehenden Ansprüche, bei welchem der gesamte offene Bereich des Films 5 bis 50% der Fläche des Films ausmacht.

12. Abdeckmaterial nach einem der vorhergehenden Ansprüche, bei welchem die Tiefe des eingedrückten Bereiches 0,2 is 3,0 mm beträgt.

13. Abdeckmaterial nach einem der vorhergehenden Ansprüche, bei welchem die eingedrückten Bereiche von unterschiedlicher Größe sind.

14. Abdeckmaterial nach einem der vorhergehenden Ansprüche, bei welchem die eingedrückten Bereiche zueinander versetzt angeordnet sind.

15. Abdeckmaterial nach einem der vorhergehenden Ansprüche, bei welchem die eingedrückten Bereiche in einer zufälligen Anordnung vorhanden sind.

16. Ein ein Abdeckmaterial nach einem der vorhergehenden Ansprüche aufweisendes absorbierendes Mittel in Verbindung mit einem absorbierenden Material.

17. Verfahren für die Herstellung eines Abdeckmaterials, dadurch gekennzeichnet, daß das Verfahren das Pressen eines Films mit einer Vielzahl von Öffnungen gegen ein Muster von erhabenen und/oder eingedrückten Bereichen beinhaltet, wodurch ein Muster von Eindrückungen in den mit Öffnungen versehenen Film eingedrückt wird, und die Anzahl der Öffnungen und Eindrückungen derart ist, daß
(i) jeder eingedrückte Bereich des Abdeckmaterials wenigstens eine Öffnung aufweist,
(ii) die Öffnungen sowohl in den eingedrückten Bereichen als auch in den Bereichen zwischen den eingedrückten Bereichen des Abdeckmaterials sind,
(iii) im wesentlichen alle eingedrückten Bereiche gekrümmte äußere Begrenzungen haben und
(iv) der Bereich zwischen den eingedrückten Bereichen des Abdeckmaterials 22 bis 50% der Fläche des Filmes ausmacht.

## Revendications

1. Élément de revêtement convenant pour être utilisé dans un dispositif absorbant qui comporte une pellicule possédant une multiplicité de zones en renfoncement, et dans lequel chaque zone en renfoncement contient au moins une ouverture, et essentiellement toutes les zones en renfoncement possèdent des pourtours incurvés, caractérisé en ce
(i) la pellicule possède un plus grand nombre d'ouvertures qu'il y a de zones en renfoncement,
(ii) les ouvertures sont situées dans les zones en renfoncement et dans les zones situées entre les zones en renfoncement, et
(iii) la surface entre les zones en renfoncement est égale à 22-50 % de la surface de la pellicule.

2. Élément de revêtement selon la revendication 1, dans lequel le périmètre de la zone en renfoncement est essentiellement circulaire ou ellipsoïdal.

3. Élément de revêtement selon la revendication 1 ou 2, dans lequel les renfoncements sont lobés.

4. Élément de revêtement selon la revendication 1, dans lequel les zones en renfoncement contiennent en moyenne plus d'une ouverture par zone en renfoncement.

5. Élément de revêtement selon l'une quelconque des revendications précédentes, dans lequel les zones en renfoncement contiennent chacune jusqu'à 8 ouvertures.

6. Élément de revêtement selon l'une des revendications précédentes, dans lequel la surface entre les zones en renfoncement constitue 25 à 40 % de la surface de la pellicule.

7. Élément de revêtement selon l'une quelconque des revendications précédentes, contenant 40 renfoncements par centimètre carré de la pellicule.

8. Élément de revêtement selon l'une quelconque des revendications précédentes, dans lequel les ouvertures sont réparties d'une manière essentiellement identique au-dessus à la fois des zones de renfoncement de la pellicule et des zones entre les renfoncements.

9. Élément de revêtement selon l'une quelconque des revendications précédentes, contenant 8 à 300 ouvertures par centimètre carré.

10. Élément de revêtement selon l'une quelconque des revendications précédentes, dans lequel chaque ouverture possède une surface comprise entre 0,01 millimètre carré et 1,0 millimètre carré.

11. Élément de revêtement selon l'une quelconque des revendications précédentes, dans lequel la surface ouverte totale de la pellicule est comprise entre 5 et 50 % de la surface de la pellicule.

12. Élément de revêtement selon l'une quelconque des revendications précédentes, dans lequel la profondeur de la zone en renfoncement est comprise entre 0,2 et 3,0 mm.

13. Élément de revêtement selon l'une quelconque des revendications précédentes, dans lequel les zones en renfoncement possèdent des tailles différentes.

14. Élément de revêtement selon l'une quelconque des revendications précédentes, dans lequel les zones en renfoncement sont disposées selon un réseau en étant réciproquement décalées.

15. Élément de revêtement selon l'une quelconque des revendications précédentes, dans lequel les zones en renfoncement sont disposées selon un réseau aléatoire.

16. Dispositif absorbant comprenant un élément de revêtement selon l'une quelconque des revendications précédentes en combinaison avec un matériau absorbant.

17. Procédé pour fabriquer un élément de revêtement, caractérisé en ce que le procédé consiste à comprimer une pellicule possédant une multiplicité d'ouvertures entre un réseau de zones surélevées et/ou en renfoncement, ce qui a pour effet qu'un réseau de renfoncement est imprimé dans la pellicule ajourée, le nombre d'ouvertures et de renfoncements étant tel que
(i) chaque zone en renfoncement de l'élément de revêtement comporte au moins une ouverture,
(ii) les ouvertures sont présentes à la fois dans les zones en renfoncement et dans les zones situées entre les zones en renfoncement de l'élément de revêtement,
(iii) essentiellement toutes les zones en renfoncement ont des pourtours incurvés, et
(iv) la surface située entre les zones en renfoncement de l'élément de revêtement est comprise entre 22 et 50 % de la surface de la pellicule.
